# EUROPEAN PATENT SPECIFICATION

(11) **EP 3 318 233 B1**
(45) Date of publication and mention of the grant of the patent: **16.12.2020**
(21) Application number: 16817549.5
(22) Date of filing: 26.04.2016
(51) Int. Cl.: A61F 13/496, A61F 13/84

(54) **PANTS-TYPE DISPOSABLE DIAPER**
HOSENARTIGE WEGWERFWINDEL
COUCHE JETABLE DE TYPE CULOTTE

(30) Priority: 30.06.2015 JP 2015131816
(43) Date of publication of application: 09.05.2018
(73) Proprietor: Unicharm Corporation, Ehime 799-0111 (JP)
(72) Inventor: FUKASAWA, Jun, Kanonji-shi Kagawa 769-1602 (JP); YOSHIOKA, Toshiyasu, Kanonji-shi Kagawa 769-1602 (JP); NAGASE, Noriko, Kanonji-shi Kagawa 769-1602 (JP)
(74) Representative: Dolleymores
(86) International application number: PCT/JP2016/063039
(87) International publication number: WO 2017/002439

(56) References cited:
- FR-A1- 2 604 867
- JP-A- S5 925 741
- JP-A- 2005 287 662
- JP-A- 2006 043 068
- JP-A- 2010 131 111
- JP-A- 2012 192 115
- US-A1- 2006 241 559

## Description

### [Technical Field]

The present invention relates to pants-type disposable diapers.

### [Background Art]

Conventionally, pants-type disposable diapers have been known as disposable diapers. For example, PTL 1 discloses a disposable diaper including: an exterior body which is composed of a front body, a crotch portion and a back body and has a pants-type shape in which one waist circumference opening and a pair of leg-circumference openings are formed; and an absorbent main body that is arranged inside the exterior body.

FIGs 3A, 3B and 3C of PTL 1 respectively show a pants-type disposable diaper in which an upper end edge thereof has a downwardly projecting form due to a cutout formed at a center portion of an upper end region of the front body in a width direction. Further, in the front body, waist-circumference elastic materials that stretch and contract along the width direction are arranged in both sides of the cutout in the width direction and in the lower side of the cutout, thereby improving the fitting property to the skin of a wearer.

### [Citation List]

### [Patent Literature]

[PTL 1] Japanese Patent Application Laid-open Publication No. 2012-192115

US 2006/241559 A1 relates to a diaper having a front portion with at least one line of weakness adapted to be torn to provide an optional umbilical feature.

### [Summary of Invention]

### [Technical Problem]

In the disposable diaper, a region (hereinafter, referred to as a "leg-circumference peripheral region") located on both sides of the absorbent main body in the width direction in a lower end region of the front body is close to the leg circumference, and thus the number of members that is layered on the front body is small so as not to block the movement of the legs. Meanwhile, in a case in which the number of members that is layered on the front body in the peripheral region of the cutout is the same as the number of members that is layered on the front body in the leg-circumference peripheral region, rigidity around the cutout becomes low. Consequently, the shape of the cutout deforms due to the contraction of a waist-circumference elastic material in the width direction and the cutout becomes less eye-catching.

The present invention has been made in view of conventional problems such as that described above and an objective thereof is to provide a pants-type disposable diaper that can maintain the shape of the cutout to allow the cutout to be easily eye-catching.

### [Solution to Problem]

The present invention provides the pants-type disposable diaper of independent claim 1. The dependent claims specify preferred but optional features.

An aspect of the invention to achieve the above advantage is a pants-type disposable diaper having an up-down direction, a front-rear direction and a width direction intersecting with the up-down direction and the front-rear direction, the pants-type disposable diaper including: an abdomen side waist part that is configured to stretch and contract along the width direction; a back side waist part; and a band-like absorbent main body that absorbs liquid, one end portion of the absorbent main body being arranged at the abdomen side waist part, another end portion of the absorbent main body being arranged at the back side waist part via a crotch, the abdomen side waist part including a cutout at a center part of an upper end region in the width direction, a number of members that is layered on the abdomen side waist part in at least a part of an edge region that includes an outer edge of the cutout being larger than a number of members that is layered on the abdomen side waist part in each of regions that are positioned on both sides of the absorbent main body in the width direction in a lower end region of the abdomen side waist part, wherein the abdomen side waist part includes: a first sheet arranged on a skin side of the wearer; a second sheet arranged on a non-skin side of the wearer; and a separate sheet that differs from the first sheet and the second sheet, the cutout is formed by penetrating the first sheet and the second sheet, and the separate sheet includes a region that overlaps with the edge region, and wherein the separate sheet is a design sheet that is arranged between the first sheet and the second sheet, a region in the separate sheet which overlaps with the edge region is colored with a color different from an outside color of the edge region.

Features of the invention other than the above will become clear by reading the description of the present specification with reference to the accompanying drawings.

### [Advantageous Effects of Invention]

According to the present invention, it is possible to maintain the shape of the cutout to allow the cutout to be easily eye-catching.

### [Brief Description of Drawings]

[FIG. 1] FIG. 1 is a schematic perspective view showing one configuration example of a diaper associated with an embodiment of the present invention.
[FIG. 2] FIG. 2 is a plan view showing an unfolded state of the diaper associated with the embodiment.
[FIG. 3] FIG. 3 is a cross-sectional schematic diagram in a case in which the diaper associated with the embodiment is cut in an up-down direction, FIG. 3A shows a state in the leg-circumference peripheral region, and FIG. 3B shows a state in the edge region and its peripheral region.
[FIG. 4] FIG. 4 is a plan view showing an unfolded state of the diaper associated with a first reference example of the present invention.
[FIG. 5] FIG. 5 is a cross-sectional schematic diagram in a case in which the diaper associated with the first reference example is cut in the up-down direction, FIG. 5A shows a state in the leg-circumference peripheral region, and FIG. 5B shows a state in the edge region and its peripheral region.
[FIG. 6] FIG. 6 is a plan view showing an unfolded state of the diaper associated with a second reference example of the present invention.
[FIG. 7] FIG. 7 is a cross-sectional schematic diagram in a case in which the diaper associated with the second reference example is cut in the up-down direction, FIG. 7A shows a state in the leg-circumference peripheral region, and FIGs. 7B to 7D show states in the edge region and its peripheral region.
[FIG. 8] FIG. 8 is a developed plan view showing an abdomenside waist part side of the diaper associated with a third reference example of the present invention.
[FIG. 9] FIG. 9 is a plan view showing an unfolded state of the diaper associated with a fourth reference example of the present invention.
[FIG. 10] FIG. 10 is a cross-sectional schematic diagram in a case in which the diaper associated with the fourth reference example is cut in the up-down direction, FIG. 10A shows a state in the leg-circumference peripheral region, and FIG. 10B shows a state in the edge region and its peripheral region.

### [Description of Embodiments]

At least the following matters will become clear with the description of this specification and the attached drawings.

Disclosed is a pants-type disposable diaper having an up-down direction, a front-rear direction and a width direction intersecting with the up-down direction and the front-rear direction, the pants-type disposable diaper including: an abdomen side waist part that is configured to stretch and contract along the width direction; a back side waist part; and a band-like absorbent main body that absorbs liquid, one end portion of the absorbent main body being arranged at the abdomen side waist part, another end portion of the absorbent main body being arranged at the back side waist part via a crotch, the abdomen side waist part including a cutout at a center part of an upper end region in the width direction, a number of members that is layered on the abdomen side waist part in at least a part of an edge region that includes an outer edge of the cutout being larger than a number of members that is layered on the abdomen side waist part in each of regions that are positioned on both sides of the absorbent main body in the width direction in a lower end region of the abdomen side waist part.

According to such a pants-type disposable diaper, the number of members that is layered on the abdomen side waist part in at least a part of the edge region including the outer edge of the cutout is made larger than the number of members that is layered on the abdomen side waist part in each of the regions positioned on both sides of the absorbent main body in the width direction in the lower end region of the abdomen side waist part, thereby enhancing rigidity. Accordingly, the contraction of the abdomen side waist part in the width direction in the edge region of the cutout is suppressed, and thus it is possible to maintain the shape of the cutout and allow the existence of the cutout to be readily recognized.

In the above pants-type disposable diaper, the abdomen side waist part includes: a first sheet arranged on a skin side of the wearer; a second sheet arranged on a non-skin side of the wearer; and a separate sheet that differs from the first sheet and the second sheet, the cutout is formed by penetrating the first sheet and the second sheet, and the separate sheet includes a region that overlaps with the edge region.

According to such a pants-type disposable diaper, the separate sheet that differs from the first sheet and the second sheet is overlapped with the edge region, thereby the number of members that is layered on the abdomen side waist part in the edge region is increased so that rigidity can be enhanced. Accordingly, the contraction of the abdomen side waist part in the width direction in the edge region is suppressed, and thus it is possible to maintain the shape of the cutout and allow the existence of the cutout to be readily recognized.

In the above pants-type disposable diaper, it is preferable that the cutout is formed by penetrating the first sheet, the second sheet and the separate sheet, and a distance from an upper end of the abdomen side waist part to an upper end of the separate sheet is less than or equal to a maximum length of the cutout in the up-down direction.

According to such a pants-type disposable diaper, since the upper end of the separate sheet is positioned between the upper end of the abdomen side waist part and the bottom of the cutout, the separate sheet is overlapped in a wider range of the edge region, so that rigidity can be further enhanced.

In the above pants-type disposable diaper, it is preferable that an upper end of the separate sheet is arranged so as to overlap with an upper end of the first sheet and an upper end of the second sheet, and a length of the separate sheet in the up-down direction is larger than a maximum length of the cutout in the up-down direction.

According to such a pants-type disposable diaper, the separate sheet has a longer length in the up-down direction than the maximum length of the cutout, and the cutout is formed by penetrating the separate sheet along with the first sheet and the second sheet. This allows the separate sheet to overlap throughout the edge region, so that rigidity in the edge region can be further enhanced.

In the above pants-type disposable diaper, the separate sheet is a design sheet that is arranged between the first sheet and the second sheet,
a region in the separate sheet which overlaps with the edge region is colored with a color different from an outside color of the edge region.

According to such a pants-type disposable diaper, by coloring the region in the separate sheet, which overlaps with the edge region, the outer edge of the cutout is made eye-catching, so that the position of the cutout can be readily recognized. Further, when a design sheet to which patterns have been applied serves as a separate sheet, a coloring process can be performed simultaneously with an application process of the patterns to the design sheet, so that it is not necessary to add a coloring process.

In the above pants-type disposable diaper, it is preferable that the one end portion of the absorbent main body includes a region that overlaps with the edge region.

According to such a pants-type disposable diaper, the one end portion of the absorbent main body is extended up to the upper side of the abdomen side waist part, and thus it is possible to enhance rigidity due to the increase of the number of members that is layered on the abdomen side waist part in the edge region. Thus, it is not necessary to separately prepare a member for enhancing rigidity in the edge region.

In the above pants-type disposable diaper, it is preferable that the absorbent main body includes: an absorbent body that absorbs and retains liquid; a top sheet arranged on a skin side with respect to the absorbent body; and a back sheet arranged on a non-skin side with respect to the absorbent body, and one end portion of the back sheet includes a region that overlaps with the edge region.

According to such a pants-type disposable diaper, the one end portion of the back sheet is extended up to the upper side of the abdomen side waist part, and thus it is possible to enhance rigidity due to the increase of the number of members that is layered on the abdomen side waist part in the edge region and to prevent, in the wider range, liquid that has been absorbed in the absorbent body from leaking out to the outside.

In the above pants-type disposable diaper, it is preferable that the absorbent main body includes: an absorbent body that absorbs and retains liquid; a top sheet arranged on a skin side with respect to the absorbent body; and a back sheet arranged on a non-skin side with respect to the absorbent body, and one end portion of the top sheet includes a region that overlaps with the edge region.

According to such a pants-type disposable diaper, the one end portion of the top sheet is extended up to the upper side of the abdomen side waist part, and thus it is possible to enhance rigidity due to the increase of the number of members that is layered on the abdomen side waist part in the edge region, and to suppress a sense of discomfort against the skin because the end portion of the back sheet does not come into contact with the skin of the wearer.

In the above pants-type disposable diaper, it is preferable that side cuff portions are provided on both sides of the absorbent main body in the width direction along the absorbent main body, and an upper end portion of each of the side cuff portions includes a region that overlaps with the edge region.

According to such a pants-type disposable diaper, the upper end portions of the side cuff portions are extended up to the upper side of the abdomen side waist part to be overlapped with the edge region, and thus rigidity is enhanced in both end sides of the edge region in the width direction. Accordingly, even if the abdomen side waist part contracts in the width direction, boundary between the cutout and regions in the abdomen side waist part which are positioned on both sides of the cutout in the width direction is maintained, thereby preventing the shape of the cutout from being easily deformed.

In the above pants-type disposable diaper, it is preferable that the absorbent main body includes: an absorbent body that absorbs and retains liquid; a top sheet arranged on a skin side with respect to the absorbent body; and a back sheet arranged on a non-skin side with respect to the absorbent body, and the side cuff portions are formed by allowing both end portions of the back sheet in the width direction to be folded back in the width direction and to be joined to the top sheet.

According to such a pants-type disposable diaper, since the side cuff portions are formed by allowing the both end portions of the back sheet in the width direction to be folded back in the width direction and to be joined to the top sheet, it is not necessary to separately provide a member for forming the side cuff portions, thereby resulting in the reduction of manufacturing cost. Further, rigidity can be enhanced in the edge region without providing a separate sheet member.

In the above pants-type disposable diaper, it is preferable that the abdomen side waist part includes a folded-back portion in which an upper end portion of the abdomen side waist part is folded back downwardly, and the folded-back portion includes a region that overlaps with the edge region.

According to such a pants-type disposable diaper, since the upper end portion of the abdomen side waist part is folded back downwardly, rigidity is enhanced in the edge region, and rigidity of the upper end portion of the abdomen side waist part is also enhanced, thereby suppressing contraction of the upper end portion of the abdomen side waist part in the width direction. Thus, the fastening to the skin when the diaper is worn can be eased.

In the above pants-type disposable diaper, it is preferable that a length of the folded-back portion which is folded back downwardly is longer than a maximum length of the cutout in the up-down direction.

According to such a pants-type disposable diaper, the length of the folded-back portion that is folded back downwardly is longer than the maximum length of the cutout in the up-down direction, and thus the folded-back portion overlaps throughout the edge region. Accordingly, rigidity in the edge region can be further enhanced.

### === Embodiment ===

Description of the pants-type disposable diaper that targets mainly newborns as wearers will be given as an example of a pants-type disposable diaper 11 (hereinafter, referred to simply as a diaper 11) associated with an embodiment of the present invention.

### <Configuration of Diaper 11>

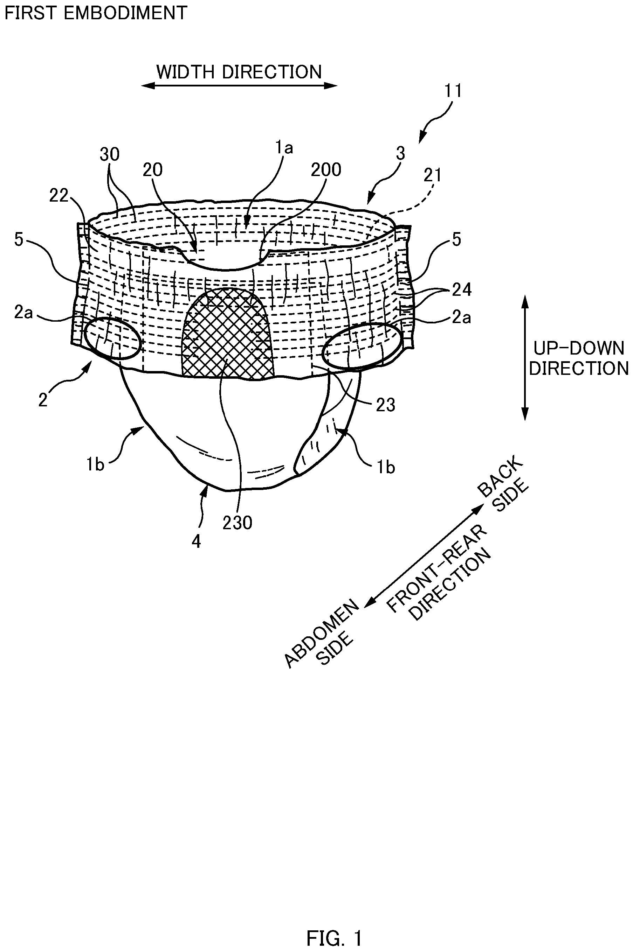

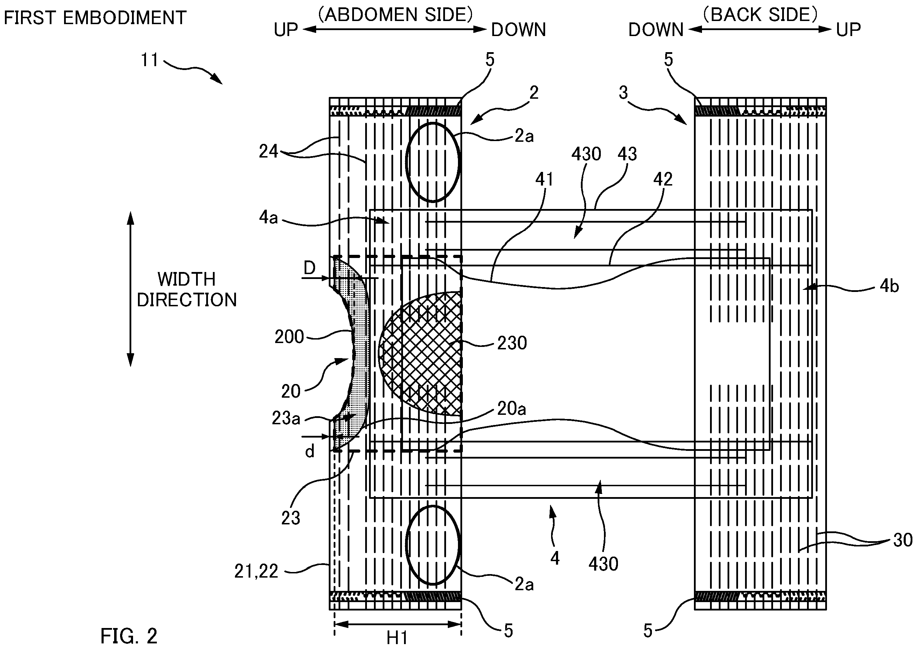

The configuration of the diaper 11 is described with reference to FIG.1 and FIG. 2.

FIG. 1 is a schematic perspective view showing one configuration example of the diaper 11 associated with the embodiment. FIG. 2 is a plan view showing an unfolded state of the diaper 11 associated with the embodiment.

The diaper 11 includes an "up-down direction" in which the waist circumference side of the wearer is referred to as an upper side (up) and the crotch side of the wearer is referred to as a lower side (down), a "front-rear direction" in which the abdomen side of the wearer is referred to as a front side (front) and the back side of the wearer is referred to as a rear side (rear), and a "width direction" that intersects with the up-down direction and the front-rear direction. Further, in a state in which the wearer wears the diaper 11, one side that comes into contact with the skin of the wearer is referred to as a "skin side", and the other side thereof is referred to as a "non-skin side".

As illustrated in FIG. 1, the diaper 11 includes an abdomen side waist part 2 that covers the abdomen side (front side) of the wearer, a back side waist part 3 that covers the back side (rear side) of the wearer, and a band-like absorbent main body 4 that absorbs liquid. A waist circumference opening 1a is formed on the upper side of the diaper 11, and leg circumference openings 1b are formed respectively on both sides of the diaper 11 in the width direction.

The abdomen side waist part 2 has, as illustrated in FIG. 2, a rectangular shape when viewed from above and a long side in the width direction. The abdomen side waist part 2 includes a first sheet 21 arranged on the skin side of the wearer, a second sheet 22 arranged on the non-skin side of the wearer, and a separate sheet 23 that differs from the first sheet 21 and the second sheet 22. The first sheet 21 and the second sheet 22 have the same size as an outer shape of the abdomen side waist part 2 and are sheet members formed with soft nonwoven fabric or the like.

The abdomen side waist part 2 includes a cutout 20 that is formed by penetrating the first sheet 21, the second sheet 22 and the separate sheet 23 at a center part of an upper end region in the width direction. This cutout 20 is for preventing the abdomen side waist part 2 from abutting against such as a navel and a clip attached to the navel cord of the newborn who is a wearer.

In the present embodiment, the cutout 20 has a curved shape in which an outer edge 200 curves toward the downward. However, the shape of the cutout 20 is not particularly limited. Further, the cutout 20 is not necessarily formed by penetrating the first sheet 21, the second sheet 22 and the separate sheet 23 and may be formed by penetrating at least the first sheet 21 and the second sheet 22.

In the abdomen side waist part 2, a plurality of abdomen side elastic strings 24 that can stretch and contract in the width direction is arranged side by side in the up-down direction in an extended state, and the first sheet 21, the second sheet 22 and the separate sheet 23 contract in the width direction accompanied by the contraction of the abdomen side elastic strings 24 in the width direction. Note that, a plurality of abdomen side elastic strings 24 is not necessarily arranged in the abdomen side waist part 2. It is sufficient that the abdomen side waist part 2 can stretch and contract in the width direction, for example, by using stretchable nonwoven fabric or the like for the first sheet 21 and the second sheet 22.

The separate sheet 23 is arranged between the first sheet 21 and the second sheet 22, and includes a region 23a that overlaps with at least a part of an edge region 20a including the outer edge 200 of the cutout 20 as illustrated in FIG. 2. In FIG. 2, the region 23a that overlaps with the edge region 20a is shown by dotted patterns. This edge region 20a is a region within a range that projects by a maximum length D of the cutout 20 in the up-down direction or within a range that projects about maximum 10 mm, from the outer edge 200 of the cutout 20 toward the downward and both sides in the width direction.

In this way, in addition to the first sheet 21 and the second sheet 22, the separate sheet 23 overlaps with at least a part of the edge region 20a, and thus the number of members that is layered on the abdomen side waist part 2 is increased in at least a part of the edge region 20a. Accordingly, it is possible to enhance rigidity in the edge region 20a.

In the present embodiment, as illustrated in FIG. 2, a distance d from the upper end of the abdomen side waist part 2 to the upper end of the separate sheet 23 is less than or equal to the maximum length D of the cutout 20 in the up-down direction (d≤D). In other words, the upper end of the separate sheet 23 is positioned between the upper end of the abdomen side waist part 2 and the bottom of the cutout 20 in the up-down direction. Thus, the separate sheet 23 includes a region 23a that is overlapped in the wider range of the edge region 20a, thereby further enhancing rigidity in the edge region 20a. FIG. 2 shows a case in which the distance d from the upper end of the abdomen side waist part 2 to the upper end of the separate sheet 23 is 0<d<D.

When the distance d from the upper end of the waist circumference portion 2 to the upper end of the separate sheet 23 is the maximum length D (d=D) of the cutout 20 in the up-down direction, the cutout 20 is formed by penetrating only the first sheet 21 and the second sheet 22 without penetrating the separate sheet 23.

Further, it is preferable that the upper end of the separate sheet 23 is arranged so as to be overlapped with the upper end of the abdomen side waist part 2 (the upper end of the first sheet 21 and the upper end of the second sheet 22) (d=0), and a length H1 of the separate sheet 23 in the up-down direction is set so as to be longer than the maximum length D of the cutout 20 in the up-down direction (H1>D). The reason is that the separate sheet 23 includes the region 23a which overlaps throughout the edge region 20a, and the rigidity in the edge region 20a can be further enhanced.

Even if the upper end of the separate sheet 23 is not arranged so as to be overlapped with the upper end of the first sheet 21 and the upper end of the second sheet 22, it is preferable that the upper end of the separate sheet 23 is positioned on the upper side than an abdomen side elastic string 24 located above out of two abdomen side elastic strings 24 which are respectively arranged on both sides of the cutout 20 in the width direction. This is for suppressing the contraction of the abdomen side waist part 2 in the width direction caused by the contraction of the two abdomen side elastic strings 24 in the width direction because it is considered that the contraction of the two abdomen side elastic strings 24 in the width direction has the greatest influence as a cause of deformation of the shape of the cutout 20.

Further, it is preferable that both ends of the separate sheet 23 in the width direction are each positioned outside of the outer edge 200 of the cutout 20 in the width direction. Accordingly, the separate sheet 23 is overlapped in a part of the edge region 20a which is positioned on both sides of the cutout 20 in the width direction, thus further enhancing rigidity of the edge region 20a.

In the present embodiment, the separate sheet 23 is a so-called design sheet to which a pattern 230 is applied. As a design sheet, a sheet formed by printing the pattern 230 on a film such as polyethylene and polypropylene can be used. The pattern 230 is shown by half-tone dot meshing in FIG. 1 and FIG. 2.

The region 23a of the separate sheet 23, which overlaps with the edge region 20a, is colored with a color different from the outside color of the edge region 20a. For example, an aspect can be exemplified such that the region 23a of the separate sheet 23, which overlaps with the edge region 20a, is colored with blue, and the outer side of the edge region 20a is not colored.

In this way, by using the difference between the color of the region 23a that overlaps with the edge region 20a and the color of the outer side of the edge region 20a, the outer edge 200 of the cutout 20 can be easily visually recognized, and the cutout 20 can be made eye-catching.

Further, the design sheet to which the pattern 230 has been applied acts as the separate sheet 23, and thus a function as a design sheet and a function as a separate sheet that enhances rigidity in the edge region 20a can be secured by one sheet member. Moreover, the region 23a that overlaps with the edge region 20a can be colored in the same process as the process of printing the pattern 230, so that it is not necessary to separately provide a process for coloring the region 23a that overlaps with the edge region 20a in order to make the cutout 20 eye-catching, and it is not required to increase manufacturing processes of the diaper 11.

The back side waist part 3 has a rectangular shape when viewed from above and a long side in the width direction, as well as the abdomen side waist part 2. The back side waist part 3 is a sheet member that is formed with such as two soft nonwoven fabric. In the present embodiment, a plurality of back side elastic strings 30 that can stretch and contract in the width direction is arranged side by side in the back side waist part 3 in the up-down direction, and two sheet members contract in the width direction accompanied by the contraction of the back side elastic strings 30 in the width direction.

As illustrated in FIG. 1, both sides of the abdomen side waist part 2 and the back side waist part 3 in the width direction are joined at a joined part 5 by fusion such as heat-sealing and sonic sealing, and by an adhesive such as hot-melt adhesive. Accordingly, the abdomen side waist part 2 and the back side waist part 3 are annually connected to form the waist circumference opening 1a.

In the absorbent main body 4, as shown in FIG. 2, one end portion 4a in the longitudinal direction is arranged in the abdomen side waist part 2, and the other end portion 4b is arranged in the back side waist part 3 via the crotch of the wearer. The absorbent main body 4 includes an absorbent body 41 that absorbs and retains liquid such as urine, a top sheet 42 arranged on the skin side with respect to the absorbent body 41, and a back sheet 43 arranged on the non-skin side with respect to the absorbent body 41.

The absorbent body 41 includes an absorbent core in which liquid absorbent materials are formed into a predetermined shape (for example, a substantially sandglass shape when viewed from above), and a core wrapping sheet that covers an outer circumferential surface of the absorbent core. As liquid absorbent materials, liquid-absorbent fiber such as pulp fiber and liquid absorbent particles such as super absorbent polymer (so-called SAP) can be exemplified. As a core wrapping sheet, a liquid-permeable sheet such as tissue paper and nonwoven fabric can be used. It is not always necessary for the absorbent body 41 to include a core wrapping sheet, and it is sufficient that the absorbent body 41 is formed by a material that can absorb liquid and retain it in the inside thereof.

The top sheet 42 is formed by a liquid-permeable soft sheet member such as air-through nonwoven fabric. The back sheet 43 includes a liquid-impermeable sheet member such as polyethylene (PE) film and polypropylene (PP) film, and a liquid-permeable sheet member such as nonwoven fabric. Note that, it is not always necessary for the back sheet 43 to include a liquid-impermeable sheet member and a liquid-permeable sheet member, and it is sufficient that, for example, the back sheet 43 may include two liquid-impermeable sheet members and may be formed only by one liquid-impermeable sheet member.

The absorbent body 41, the top sheet 42 and the back sheet 43 are each joined to adjacent members in the thickness direction with adhesive such as hot-melt adhesive. As an application pattern of the adhesive, a Ω pattern, a spiral pattern, a stripe pattern or the like can be exemplified. This also applies to an application pattern of the other adhesive.

Side cuff portions 430 are provided along the absorbent main body 4 on both sides of the absorbent main body 4 in the width direction. These side cuff portions 430 are each for preventing such as urine from leaking out to the outside from the leg circumference openings 1b (see FIG. 1). In the present embodiment, the side cuff portions 430 are formed by allowing both end portions of the back sheet 43 in the width direction to be folded back in the width direction and to be joined to the top sheet 42.

### <Regarding the number of members that is layered on the abdomen side waist part 2>

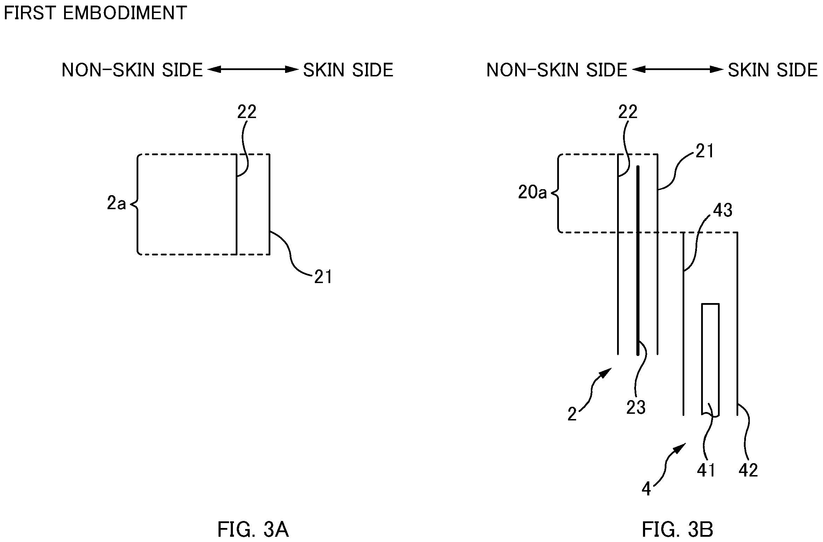

Next, the number of members that is layered on the abdomen side waist part 2 will be described with reference to FIG. 3A and FIG. 3B.

FIG. 3A and FIG. 3B are cross-sectional schematic diagrams in a case in which the diaper 11 is cut in the up-down direction. FIG. 3A shows a state in leg-circumference peripheral regions 2a, and FIG. 3B shows a state in the edge region 20a and its peripheral region.

Here, the "leg-circumference peripheral regions 2a" are referred to regions in the lower end region of the abdomen side waist part 2, which are each positioned on both sides of the absorbent main body 4 in the width direction (see FIG. 1 and FIG. 2). When the diaper 11 is worn, the leg-circumference peripheral regions 2a are regions close to the leg circumference of the wearer, and the number of members that is layered on the abdomen side waist part 2 is small so as not to block the movement of the wearer. Specifically, as illustrated in FIG. 3A, members that are layered on the abdomen side waist part 2 in each of the leg-circumference peripheral regions 2a are the first sheet 21 and the second sheet 22. In other words, the number of members that is layered on the abdomen side waist part 2 in each of the leg-circumference peripheral regions 2a is two.

Meanwhile, as illustrated in FIG. 3B, members that are layered on the abdomen side waist part 2 in at least a part of the edge region 20a are the first sheet 21, the second sheet 22 and the separate sheet 23 (design sheet). That is, the number of members that is layered on the abdomen side waist part 2 in the edge region 20a is three. Thus, the number of members that is layered on the abdomen side waist part 2 in at least a part of the edge region 20a is larger than the number of members that is layered on the abdomen side waist part 2 in each of the leg-circumference peripheral regions 2a. In FIG. 3B, the separate sheet 23 is shown by a bold line.

If the number of members that is layered on the abdomen side waist part 2 in the edge region 20a is made the same as the number of members that is layered on the abdomen side waist part 2 in each of the leg-circumference peripheral regions 2a, rigidity becomes insufficient. Also, the first sheet 21 and the second sheet 22 contract in the width direction (the abdomen side waist part 2 contracts in the width direction) accompanied by the contraction of the abdomen side elastic strings 24 in the width direction which are arranged on the both sides and the lower side of the cutout 20 in the width direction, and thus the shape of the cutout 20 is deformed, so that the cutout 20 is hardly made eye-catching.

Then, as in the present embodiment, the number of members that is layered on the abdomen side waist part 2 in at least a part of the edge region 20a is made larger than the number of members that is layered on the abdomen side waist part 2 in each of the leg-circumference peripheral regions 2a to enhance rigidity, and thus the contraction of the abdomen side waist part 2 in the width direction in the edge region 20a is suppressed. Accordingly, the shape of the cutout 20 is maintained, and it is possible to make the cutout 20 eye-catching.

In the present embodiment, a case has been described in which the separate sheet 23 including the region 23a that overlaps with at least a part of the edge region 20a is used for enhancing rigidity in the edge region 20a, and the design sheet arranged between the first sheet 21 and second sheet 22 of the abdomen side waist part 2 is applied as the separate sheet 23. Other several aspects of the diaper 11 in which the rigidity in the edge region 20a is enhanced are described for reference. In the following, respective aspects in a first reference example to a fourth reference example will be described. These reference examples fall outside of the scope of the claimed invention.

### === First reference example ===

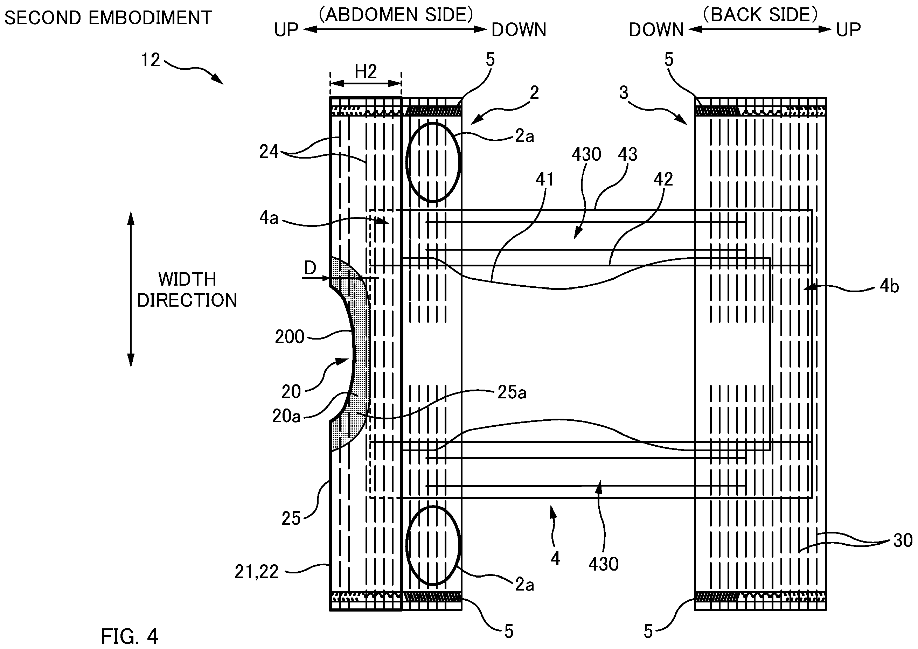

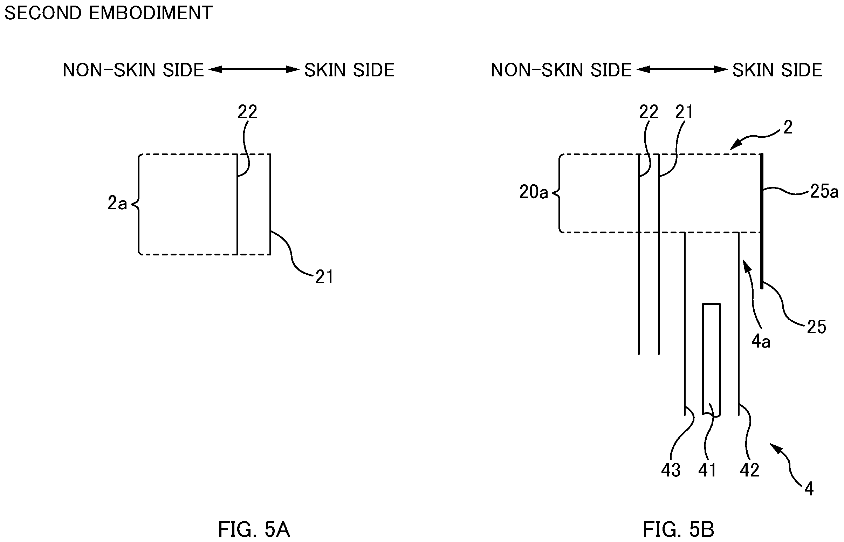

Next, the configuration of a diaper 12 associated with the first reference example in the present invention will be described with reference to FIG. 4 and FIG. 5.

In FIG. 4 and FIG. 5, the structure that is common to the structure of the diaper 11 associated with the embodiment will be indicated with the same reference numerals and explanations thereof will be omitted. It should be noted that, the same applies to a second reference example to a fourth reference example described below.

FIG. 4 is a plan view showing an unfolded state of the diaper 12 associated with the first reference example. FIG. 5A and FIG. 5B are cross-sectional schematic diagrams in a case in which the diaper 12 associated with the first reference example is cut in the up-down direction, FIG. 5A show a state in the leg-circumference peripheral region 2a, and FIG. 5B shows a state in the edge region 20a and its peripheral region.

In the diaper 12 associated with the present reference example, the configuration of a separate sheet 25 of the abdomen side waist part 2 differs from the configuration of the separate sheet 23 of the diaper 11 associated with the embodiment. The separate sheet 25 is a supplemental sheet that is arranged so as to allow one end portion 4a of the absorbent main body 4 in the longitudinal direction to be sandwiched between the separate sheet 25 and the first sheet 21 as shown in FIG. 4 and FIG. 5B. This "supplemental sheet" is for suppressing a sense of discomfort due to the abutment of the one end portion 4a of the absorbent main body 4 against the skin of the wearer by covering the one end portion 4a of the absorbent main body 4 from the skin side and for allowing the absorbent core not to come out from the one end portion 4a of the absorbent main body 4 and not to be touched with the skin.

Specifically, as shown in FIG. 4, the separate sheet 25 has a length in the width direction, which is the same as the length of the first sheet 21 and the second sheet 22 in the width direction, and the lower end portion of the separate sheet 25 is set so as to overlap with the one end portion 4a of the absorbent main body 4 (the top sheet 42 and the back sheet 43). Thus, a length H2 of the separate sheet 25 in the up-down direction is longer than the maximum length D of the cutout 20 in the up-down direction (H2 > D).

As illustrated in FIG. 5A, also in the diaper 12 associated with the present reference example, members that are layered on the abdomen side waist part 2 in each of the leg-circumference peripheral regions 2a are two sheets which are the first sheet 21 and the second sheet 22, as well as the diaper 11 associated with the embodiment.

Meanwhile, as illustrated in FIG. 5B, members that are layered on the abdomen side waist part 2 in at least a part of the edge region 20a are three sheets that are the first sheet 21, the second sheet 22, and the separate sheet 25 (supplemental sheet). Thus, the number of members that is layered on the abdomen side waist part 2 in at least a part of the edge region 20a is larger than the number of members that is layered on the abdomen side waist part 2 in each of the leg-circumference peripheral regions 2a, thereby, it is possible to enhance rigidity in the edge region 20a. In FIG. 5B, the separate sheet 25 is shown by a bold line.

In FIG. 4 and FIG. 5B, the separate sheet 25 is arranged such that the upper end of the separate sheet 25 overlaps with the upper end of the abdomen side waist part 2. However, the invention is not limited thereto, and the separate sheet 25 may be arranged so as to overlap with at least a part of the edge region 20a.

In this way, when the supplemental sheet arranged on the skin side so as to sandwich the one end portion 4a of the absorbent main body 4 between the first sheet 21 and the supplemental sheet acts as the separate sheet 25, a sense of discomfort due to the abutment of the one end portion 4a of the absorbent main body 4 against the skin of the wearer can be suppressed, and rigidity in the edge region 20a can be enhanced.

=== Second reference example ===

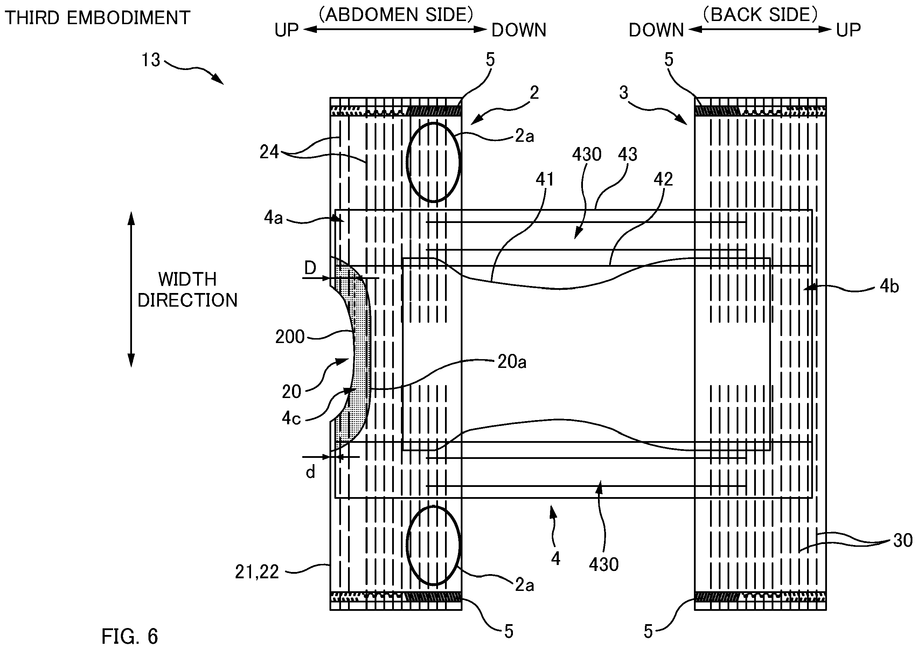

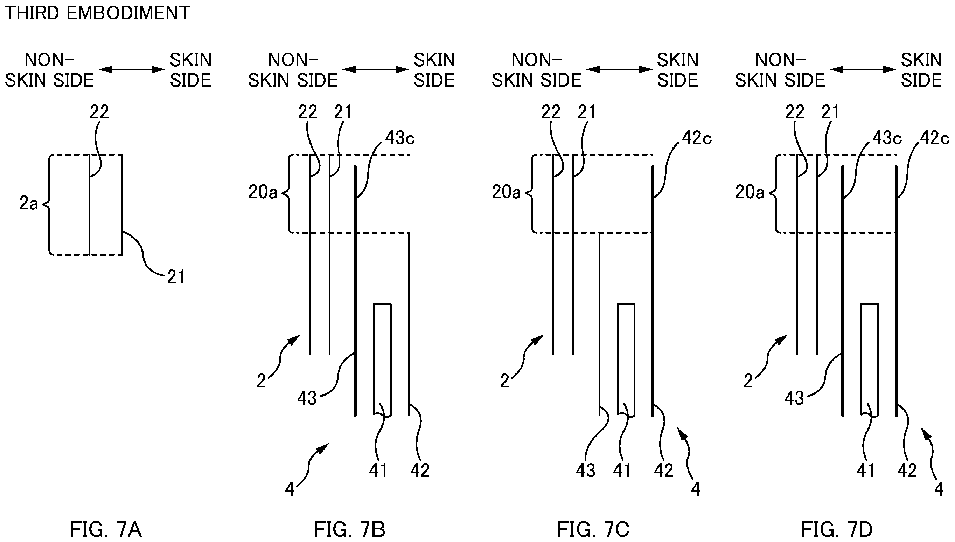

Next, the configuration of a diaper 13 associated with a second reference example of the present invention will be described with reference to FIG. 6 and FIGs. 7A to 7D.

FIG. 6 is a plan view showing an unfolded state of the diaper 13 associated with the second reference example. FIGs. 7A to 7D are cross-sectional schematic diagrams in a case in which the diaper 13 associated with the second reference example is cut in the up-down direction, FIG. 7A shows a state in the leg-circumference peripheral region 2a, and FIGs. 7B to 7D show states in the edge region 20a and its peripheral region.

In the diaper 13 associated with the present reference example, as illustrated in FIG. 6, the one end portion 4a of the absorbent main body 4 extends to the upper end side of the abdomen side waist part 2, and a region 4c that overlaps with the edge region 20a is included. Note that, a case in which the upper end in the one end portion 4a side overlaps with the upper end of the abdomen side waist part 2 is further acceptable. That is because the one end portion 4a includes the region 4c that overlaps throughout the edge region 20a and rigidity in the edge region 20a can be further enhanced.

In this way, the one end portion 4a of the absorbent main body 4 is extended to the upper end side of the abdomen side waist part 2, and thus rigidity in the edge region 20a can be enhanced due to the increase of the number of members that is layered on the abdomen side waist part 2 in at least a part of the edge region 20a. Accordingly, it is not required to separately prepare members for enhancing rigidity in the edge region 20a, thereby resulting in cost reduction.

Note that, as for the configuration in which the one end portion 4a of the absorbent main body 4 is extended up to the upper end side of the abdomen side waist part 2, several aspects can be considered.

First, as illustrated in FIG. 7B, a case can be considered in which one end portion of the back sheet 43 of the absorbent main body 4 includes a region 43c that overlaps with at least a part of the edge region 20a. In FIG. 7B, the back sheet 43 is shown by a bold line.

In this case, as illustrated in FIG. 7A, members that are layered on the waist circumference portion 2 in the leg-circumference peripheral region 2a are two sheets that are the first sheet 21 and the second sheet 22, whereas members that are layered on the waist circumference portion 2 in at least a part of the edge region 20a are three sheets that are the first sheet 21, the second sheet 22 and the back sheet 43. Thus, the number of members that is layered on the abdomen side waist part 2 in at least a part of the edge region 20a is larger than the number of members that is layered on the abdomen side waist part 2 in the leg-circumference peripheral region 2a. Thereby, rigidity in the edge region 20a can be enhanced.

When the back sheet 43 is formed by only one sheet member, the number of members that is layered on the abdomen side waist part 2 in at least a part of the edge region 20a is three. Meanwhile, for example, when the back sheet 43 is formed by two sheet members, the number of members that is layered on the abdomen side waist part 2 in at least a part of the edge region 20a is four, and thus rigidity in the edge region 20a can be further enhanced.

In this way, the one end portion of the back sheet 43 of the absorbent main body 4 is extended up to the upper end side of the abdomen side waist part 2, and thus it is possible to enhance rigidity in the edge region 20a and to prevent, in a wider range, liquid such as urine that has been absorbed in the absorbent body 41 from leaking out to the outside.

Next, as illustrated in FIG. 7C, a case can be considered in which one end portion of the top sheet 42 of the absorbent main body 4 includes a region 42c that overlaps with at least a part of the edge region 20a. In FIG. 7C, the top sheet 42 is shown by a bold line.

Also in this case, as illustrated in FIG. 7A, members that are layered on the waist part 2 in the leg-circumference peripheral region 2a are two sheets that are the first sheet 21 and the second sheet 22, whereas members that are layered on the waist part 2 in at least a part of the edge region 20a are three sheets that are the first sheet 21, the second sheet 22 and the top sheet 42. Thus, the number of members that is layered on the abdomen side waist part 2 in at least a part of the edge region 20a is larger than the number of members that is layered on the abdomen side waist part 2 in the leg-circumference peripheral region 2a. Accordingly, it is possible to enhance rigidity in the edge region 20a.

In this way, the one end portion of the top sheet 42 of the absorbent main body 4 is extended up to the upper end side of the abdomen side waist part 2, and thus it is possible to enhance rigidity in the edge region 20a and to suppress a sense of discomfort with respect to the skin because the one end portion of the back sheet 43 including a liquid-impermeable sheet member that is harder than the top sheet 42 does not directly come into contact with the skin of the wearer.

As illustrated in FIG. 7D, a case can be considered in which the one end portion of the top sheet 42 of the absorbent main body 4 includes a region 42c that overlaps with at least a part of the edge region 20a, and the one end portion of the back sheet 43 includes a region 43c that overlaps with at least a part of the edge region 20a. In FIG. 7D, the top sheet 42 and the back sheet 43 are shown by bold lines.

In this case, as illustrated in FIG. 7A, members that are layered on the waist circumference portion 2 in the leg-circumference peripheral region 2a are two sheets that are the first sheet 21 and the second sheet 22, whereas members that are layered on the waist circumference portion 2 in at least a part of the edge region 20a are four sheets that are the first sheet 21, the second sheet 22, the top sheet 42 and the back sheet 43. Accordingly, the number of members that is layered on the abdomen side waist part 2 in at least a part of the edge region 20a is larger than the number of members that is layered on the abdomen side waist part 2 in the leg-circumference peripheral region 2a, and thus rigidity in the edge region 20a can be further enhanced.

### === Third reference example ===

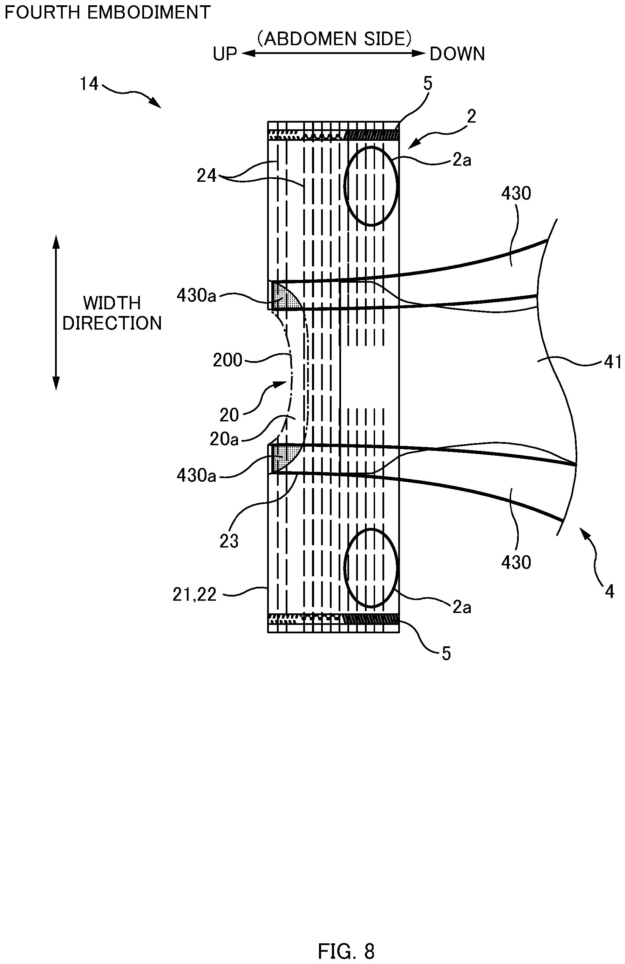

Next, the configuration of a diaper 14 associated with a third reference example of the present invention is described with reference to FIG. 8.

FIG. 8 is a developed plan view showing the abdomen side waist part 2 side of the diaper 14 associated with the third reference example.

The diaper 14 associated with the present reference example, as illustrated in FIG. 8, includes regions 430a in which upper end portions of side cuff portions 430 that are respectively provided in both sides of the absorbent main body 4 in the width direction overlap with the edge region 20a. Specifically, the regions 430a in which the upper end portions of the side cuff portions 430 overlap with the edge region 20a are parts of the edge region 20a, which are positioned on both sides of the cutout 20 in the width direction. In FIG. 8, the edge region 20a is shown by single-dotted chain lines, and the side cuff portions 430 are shown by bold lines, respectively. In addition, the regions 430a in which the upper end portions of the side cuff portions 430 overlap with the edge region 20a are shown by dotted patterns.

In this way, the upper end portions of the side cuff portions 430 are extended up to the upper end side of the abdomen side waist part 2 to overlap with the edge region 20a, and thus rigidity is enhanced in both ends side of the edge region 20a in the width direction. Also, even when the abdomen side waist part 2 contracts in the width direction accompanied by the contraction of the abdomen side elastic strings 24, parts of the outer edge 200 of the cutout 20 which are positioned on both sides in the width direction (boundaries between the cutout 20 and regions of the abdomen side waist part 2 that are positioned on both sides of the cutout 20 in the width direction) are maintained, and thereby the shape of the cutout 20 is prevented from being deformed and is easily maintained.

Note that, it is preferable that the side cuff portions 430 are formed so as to extend up to the position where the upper ends thereof overlap with the upper end of the abdomen side waist part 2. The reason is that, such side cuff portions 430 include, in a wider range, the regions 430a in which the upper end portions of the side cuff portions 430 overlap with the edge region 20a, so that rigidity is further enhanced in both ends side of the edge region 20a in the width direction.

Further, in the present reference example, as described in the embodiment, since the side cuff portions 430 are formed by allowing the both ends portions of the back sheet 43 of the absorbent main body 4 in the width direction to be folded back to the inside in the width direction and to be joined to the top sheet 42, rigidity in the edge region 20a can be enhanced without providing a separate sheet member. Although it is preferable that the side cuff portions 430 are formed using the back sheet 43, the invention is not always limited thereto. A separate member may be joined to the absorbent main body 4.

### === Fourth reference example ===

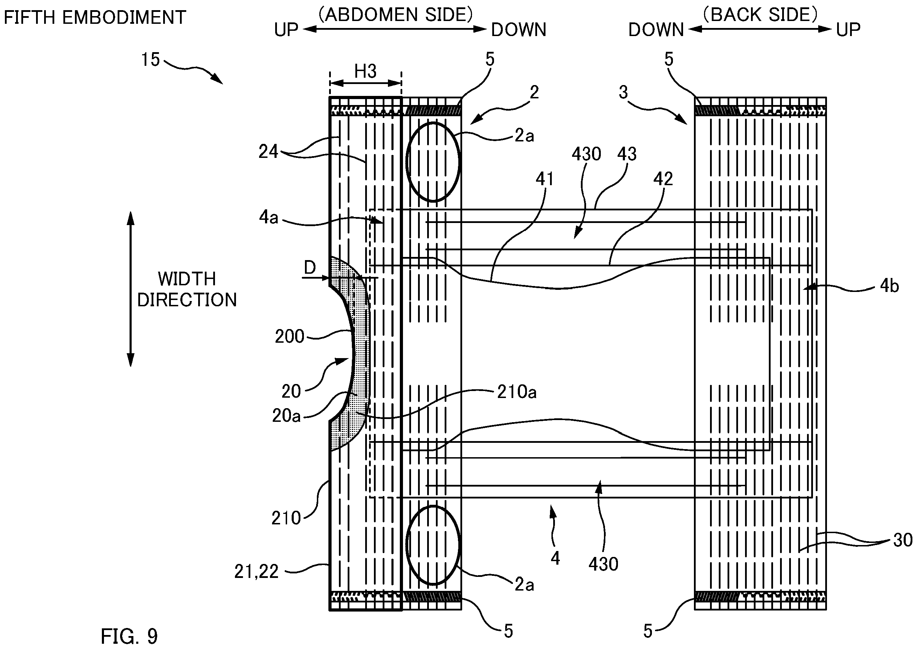

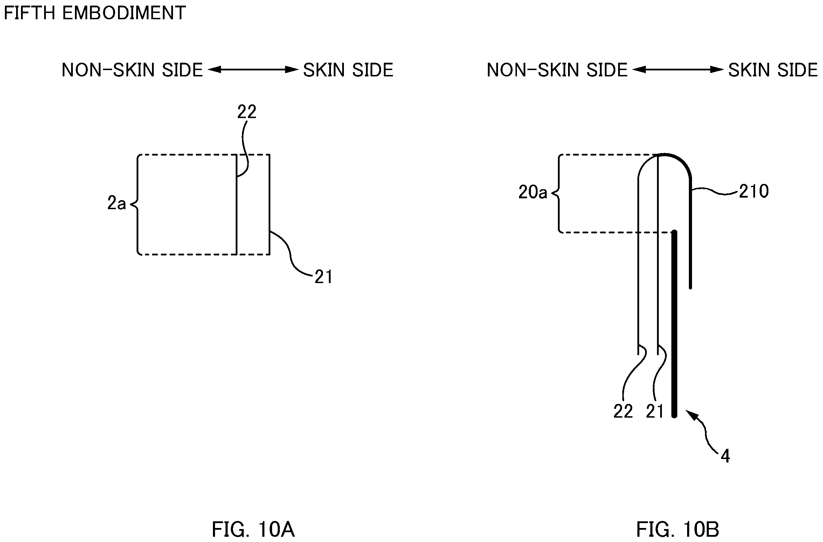

Next, the configuration of a diaper 15 associated with a fourth reference example of the present invention is described with reference to FIG. 9, FIG. 10A and FIG. 10B.

FIG. 9 is a plan view showing an unfolded state of the diaper 15 associated with the fourth reference example. FIG. 10A and FIG. 10B are cross-sectional schematic diagrams in a case in which the diaper 15 associated with the fourth reference example is cut in the up-down direction, FIG. 10A shows a state in the leg-circumference peripheral region 2a, and FIG. 10B shows a state in the edge region 20a and its peripheral region.

The abdomen side waist part 2 of the diaper 15 associated with the present reference example includes a folded-back portion 210 in which an upper end portion of the abdomen side waist part 2 is folded-back downwardly as illustrated in FIG. 9 and FIG. 10B. In FIG. 9 and FIG. 10B, the folded-back portion 210 is shown by a bold line. The folded-back portion 210 includes a region 210a that overlaps with the edge region 20a.

Thus, rigidity is enhanced in the edge region 20a, and also rigidity of the upper end portion of the abdomen side waist part 2 is enhanced, thereby suppressing contraction of the upper end portion of the abdomen side waist part 2 in the width direction accompanied by the contraction of the abdomen side elastic strings 24. Accordingly, when the diaper 15 is worn, the fastening of the abdomen side waist part 2 onto the skin can be eased.

In the present reference example, the folded-back portion 210 is set so that a length H3 in the up-down direction is overlapped with the one end portion 4a of the absorbent main body 4 (the top sheet 42 and the back sheet 43). In other words, the length H3 of the folded-back portion 210, which has been folded back downwardly, is longer than the maximum length D of the cutout 20 in the up-down direction (H3>D). Accordingly, the folded-back portion 210 overlaps throughout the edge region 20a, so that rigidity in the edge region 20a can be further enhanced.

### === Others ===

The foregoing embodiment is intended to facilitate the understanding of the present invention, and the present invention is not to be construed as being limited to the embodiment.

Although the embodiment and the first to the fourth reference examples have been described as aspects that enhance rigidity in the edge region 20a, combination of the embodiment and the respective reference examples may also be applicable to the diaper. For example, when the embodiment and the second reference example are combined, a design sheet as the separate sheet 23 and the one end portion 4a of the absorbent main body 4 are arranged so as to be overlapped in the edge region 20a. In this case, the number of members that is layered on the abdomen side waist part 2 in the edge region 20a is further increased, and the rigidity in the edge region 20a can be further enhanced.

### [Reference Signs List]

2 ... abdomen side waist part
2a ... leg-circumference peripheral regions (regions that are each positioned on both sides of the absorbent main body in the width direction in the lower end region of the abdomen side waist part)
3 ... back side waist part
4 ... absorbent main body
4a ... one end portion
4b ... the other end portion
11, 12, 13, 14, 15 ... diaper (pants-type disposable diaper)
20 ... cutout
20a ... edge region
21 ... first sheet
22 ... second sheet
23 ... separate sheet, design sheet
25 ... separate sheet, supplemental sheet
41 ... absorbent body
42 ... top sheet
43 ... back sheet
200 ... outer edge
210 ... folded-back portion
430 ... side cuff portions

## Claims

1. A pants-type disposable diaper (11) having an up-down direction, a front-rear direction and a width direction intersecting with the up-down direction and the front-rear direction, the pants-type disposable diaper (11) comprising:
an abdomen side waist part (2) that is configured to stretch and contract along the width direction;
a back side waist part (3); and
a band-like absorbent main body (4) that absorbs liquid,
one end portion (4a) of the absorbent main body (4) being arranged at the abdomen side waist part (2), another end portion (4b) of the absorbent main body (4) being arranged at the back side waist part (3) via a crotch,
the abdomen side waist part (2) including a cutout (20) at a center part of an upper end region in the width direction,
a number of members that is layered on the abdomen side waist part (2) in at least a part of an edge region (20a) that includes an outer edge (200) of the cutout (20) being larger than a number of members that is layered on the abdomen side waist part (2) in each of regions (2a) that are positioned on both sides of the absorbent main body (4) in the width direction in a lower end region of the abdomen side waist part (2), wherein
the abdomen side waist part (2) includes:
a first sheet (21) arranged on a skin side of the wearer;
a second sheet (22) arranged on a non-skin side of the wearer; and
a separate sheet (23) that differs from the first sheet (21) and the second sheet (22),
the cutout (20) is formed by penetrating the first sheet (21) and the second sheet (22), and
the separate sheet (23) includes a region that overlaps with the edge region (20a), and wherein
the separate sheet (23) is a design sheet that is arranged between the first sheet (21) and the second sheet (22), and
a region (23a) in the separate sheet (23) which overlaps with the edge region (20a) is colored with a color different from an outside color of the edge region (20a).

2. The pants-type disposable diaper (11) according to claim 1, wherein
the cutout (20) is formed by penetrating the first sheet (21), the second sheet (22) and the separate sheet (23), and
a distance (d) from an upper end of the abdomen side waist part (2) to an upper end of the separate sheet (23) is less than or equal to a maximum length (D) of the cutout (20) in the up-down direction.

3. The pants-type disposable diaper (11) according to claim 2, wherein
an upper end of the separate sheet (23) is arranged so as to overlap with an upper end of the first sheet (21) and an upper end of the second sheet (22), and a length (H1) of the separate sheet (23) in the up-down direction is larger than a maximum length (D) of the cutout (20) in the up-down direction.

4. The pants-type disposable diaper (11) according to any one of claims 1 to 3, wherein
the one end portion (4a) of the absorbent main body (4) includes a region (42c, 43c) that overlaps with the edge region (20a) .

5. The pants-type disposable diaper (11) according to claim 4, wherein
the absorbent main body (4) includes:
an absorbent body (41) that absorbs and retains liquid;
a top sheet (42) arranged on a skin side with respect to the absorbent body (41); and
a back sheet (43) arranged on a non-skin side with respect to the absorbent body (41), and
one end portion of the back sheet (43) includes a region (43c) that overlaps with the edge region (20a).

6. The pants-type disposable diaper (11) according to claim 4 or 5, wherein
the absorbent main body (4) includes:
an absorbent body (41) that absorbs and retains liquid;
a top sheet (42) arranged on a skin side with respect to the absorbent body (41); and
a back sheet (43) arranged on a non-skin side with respect to the absorbent body (41), and
one end portion of the top sheet (42) includes a region (42c) that overlaps with the edge region (20a).

7. The pants-type disposable diaper (11) according to any one of claims 1 to 6, wherein,
side cuff portions (430) are provided on both sides of the absorbent main body (4) in the width direction along the absorbent main body (4), and
an upper end portion of each of the side cuff portions (430) includes a region (430a) that overlaps with the edge region (20a).

8. The pants-type disposable diaper (11) according to claim 7, wherein
the absorbent main body (4) includes:
an absorbent body (41) that absorbs and retains liquid;
a top sheet (42) arranged on a skin side with respect to the absorbent body (41); and
a back sheet (43) arranged on a non-skin side with respect to the absorbent body (41), and
the side cuff portions (430) are formed by allowing both end portions of the back sheet (43) in the width direction to be folded back in the width direction and to be joined to the top sheet (42).

9. The pants-type disposable diaper (11) according to any one of claims 1 to 8, wherein
the abdomen side waist part (2) includes a folded-back portion (210) in which an upper end portion of the abdomen side waist part (2) is folded back downwardly, and
the folded-back portion (210) includes a region that overlaps with the edge region (20a).

10. The pants-type disposable diaper (11) according to claim 9, wherein
a length of the folded-back portion (210) which is folded back downwardly is longer than a maximum length (D) of the cutout (20) in the up-down direction.

## Patentansprüche

1. Höschenähnliche Einwegwindel (11), die Folgendes aufweist: eine Oben-unten-Richtung, eine Vorn-hinten-Richtung und eine Breitenrichtung, die die Oben-unten-Richtung und die Vorn-hinten-Richtung schneidet, wobei die höschenähnliche Einwegwindel (11) Folgendes umfasst:
ein Bauchseitentaillenteil (2), das zum Dehnen und Zusammenziehen entlang der Breitenrichtung konfiguriert ist;
ein Rückseitentaillenteil (3); und
einen bandartigen absorbierenden Hauptkörper (4), der Flüssigkeit absorbiert,
wobei ein Endabschnitt (4a) des absorbierenden Hauptkörpers (4) an dem Bauchseitentaillenteil (2) angeordnet ist, ein anderer Endabschnitt (4b) des absorbierenden Hauptkörpers (4) an dem Rückseitentaillenteil (3) über einen Schrittbereich angeordnet ist,
das Bauchseitentaillenteil (2) einen Ausschnitt (20) an einem Mittelteil eines oberen Endbereichs in der Breitenrichtung einschließt,
eine Anzahl von Elementen, die auf dem Bauchseitentaillenteil (2) in mindestens einem Teil eines Randbereichs (20a) geschichtet ist, der einen Außenrand (200) des Ausschnitts (20) einschließt, größer ist als eine Anzahl von Elementen, die auf dem Bauchseitentaillenteil (2) in jedem der Bereiche (2a) geschichtet ist, die auf beiden Seiten des absorbierenden Hauptkörpers (4) in der Breitenrichtung in einem unteren Endbereich des Bauchseitentaillenteils (2) positioniert sind, wobei
das Bauchseitentaillenteil (2) Folgendes einschließt:
eine erste Lage (21), die auf einer Hautseite des Trägers angeordnet ist;
eine zweite Lage (22), die auf einer Nicht-Hautseite des Trägers angeordnet ist; und
eine separate Lage (23), die sich von der ersten Lage (21) und der zweiten Lage (22) unterscheidet,
der Ausschnitt (20) durch Durchdringen der ersten Lage (21) und der zweiten Lage (22) ausgebildet ist und
die separate Lage (23) einen Bereich einschließt, der mit dem Randbereich (20a) überlappt, und wobei
die separate Lage (23) eine Designlage ist, die zwischen der ersten Lage (21) und der zweiten Lage (22) angeordnet ist, und
ein Bereich (23a) in der separaten Lage (23), der mit dem Randbereich (20a) überlappt, farbig in einer Farbe ist, die sich von einer Außenseitenfarbe des Randbereichs (20a) unterscheidet.

2. Höschenähnliche Einwegwindel (11) nach Anspruch 1, wobei
der Ausschnitt (20) durch Durchdringen der ersten Lage (21), der zweiten Lage (22) und der separaten Lage (23) ausgebildet ist und
ein Abstand (d) von einem oberen Ende des Bauchseitentaillenteils (2) zu einem oberen Ende der separaten Lage (23) kleiner ist als oder gleich einer maximalen Länge (D) des Ausschnitts (20) in der Oben-unten-Richtung.

3. Höschenähnliche Einwegwindel (11) nach Anspruch 2, wobei
ein oberes Ende der separaten Lage (23) derart angeordnet ist, dass es mit einem oberen Ende der ersten Lage (21) und einem oberen Ende der zweiten Lage (22) überlappt, und eine Länge (H1) der separaten Lage (23) in der Oben-unten-Richtung größer ist als eine maximale Länge (D) des Ausschnitts (20) in der Oben-unten-Richtung.

4. Höschenähnliche Einwegwindel (11) nach einem der Ansprüche 1 bis 3, wobei
der eine Endabschnitt (4a) des absorbierenden Hauptkörpers (4) einen Bereich (42c, 43c) einschließt, der mit dem Randbereich (20a) überlappt.

5. Höschenähnliche Einwegwindel (11) nach Anspruch 4, wobei
der absorbierende Hauptkörper (4) Folgendes einschließt:
einen Saugkörper (41), der Flüssigkeit absorbiert und zurückhält;
eine obere Lage (42), die auf einer Hautseite in Bezug auf den Saugkörper (41) angeordnet ist; und
eine hintere Lage (43), die auf einer Nicht-Hautseite in Bezug auf den Saugkörper (41) angeordnet ist, und
ein Endabschnitt der hinteren Lage (43) einen Bereich (43c) einschließt, der mit dem Randbereich (20a) überlappt.

6. Höschenähnliche Einwegwindel (11) nach Anspruch 4 oder 5, wobei
der absorbierende Hauptkörper (4) Folgendes einschließt:
einen Saugkörper (41), der Flüssigkeit absorbiert und zurückhält;
eine obere Lage (42), die auf einer Hautseite in Bezug auf den Saugkörper (41) angeordnet ist; und
eine hintere Lage (43), die auf einer Nicht-Hautseite in Bezug auf den Saugkörper (41) angeordnet ist, und
ein Endabschnitt der oberen Lage (42) einen Bereich (42c) einschließt, der mit dem Randbereich (20a) überlappt.

7. Höschenähnliche Einwegwindel (11) nach einem der Ansprüche 1 bis 6, wobei
Seitenbündchenabschnitte (430) auf beiden Seiten des absorbierenden Hauptkörpers (4) in der Breitenrichtung entlang des absorbierenden Hauptkörpers (4) bereitgestellt sind und
ein oberer Endabschnitt von jedem der Seitenbündchenabschnitte (430) einen Bereich (430a) einschließt, der mit dem Randbereich (20a) überlappt.

8. Höschenähnliche Einwegwindel (11) nach Anspruch 7, wobei
der absorbierende Hauptkörper (4) Folgendes einschließt:
einen Saugkörper (41), der Flüssigkeit absorbiert und zurückhält;
eine obere Lage (42), die auf einer Hautseite in Bezug auf den Saugkörper (41) angeordnet ist; und
eine hintere Lage (43), die auf einer Nicht-Hautseite in Bezug auf den Saugkörper (41) angeordnet ist, und
die Seitenbündchenabschnitte (430) ausgebildet werden, in dem ermöglicht wird, dass beide Endabschnitte der hinteren Lage (43) in der Breitenrichtung zurückgefaltet werden in der Breitenrichtung und mit der oberen Lage (42) verbunden werden.

9. Höschenähnliche Einwegwindel (11) nach einem der Ansprüche 1 bis 8, wobei
das Bauchseitentaillenteil (2) einen zurückgefalteten Abschnitt (210) einschließt, in dem ein oberer Endabschnitt des Bauchseitentaillenteils (2) nach unten zurückgefaltet ist, und
der zurückgefaltete Abschnitt (210) einen Bereich einschließt, der mit dem Randbereich (20a) überlappt.

10. Höschenähnliche Einwegwindel (11) nach Anspruch 9, wobei
eine Länge des zurückgefalteten Abschnitts (210), der nach unten zurückgefaltet ist, länger ist als eine maximale Länge (D) des Ausschnitts (20) in der Oben-unten-Richtung.

## Revendications

1. Couche jetable de type culotte (11) ayant une direction allant de haut en bas, une direction allant dans le sens avant-arrière et une direction allant dans le sens de la largeur croisant la direction allant de haut en bas et la direction allant dans le sens avant-arrière, la couche jetable de type culotte (11) comportant :
une partie au niveau de la taille côté abdomen (2) qui est configurée à des fins d'étirement et de contraction le long de la direction allant dans le sens de la largeur ;
une partie au niveau de la taille côté dos (3) ; et
un corps principal absorbant similaire à une bande (4) qui absorbe du liquide,
une partie d'extrémité (4a) du corps principal absorbant (4) étant agencée au niveau de la partie au niveau de la taille côté abdomen (2), une autre partie d'extrémité (4b) du corps principal absorbant (4) étant agencée au niveau de la partie au niveau de la taille côté dos (3) par le biais d'un entrejambe,
la partie au niveau de la taille côté abdomen (2) comprenant une découpe (20) au niveau d'une partie centrale d'une région d'extrémité supérieure dans la direction allant dans le sens de la largeur,
un nombre d'éléments qui sont mis en couches sur la partie au niveau de la taille côté abdomen (2) dans au moins une partie d'une région de bord (20a) qui comprend un bord extérieur (200) de la découpe (20) étant supérieur à un nombre d'éléments qui sont mis en couche sur la partie au niveau de la taille côté abdomen (2) dans chacune des régions (2a) qui sont positionnées des deux côtés du corps principal absorbant (4) dans la direction allant dans le sens de la largeur dans une région d'extrémité inférieure de la partie au niveau de la taille côté abdomen (2), dans laquelle
la partie au niveau de la taille côté abdomen (2) comprend :
une première feuille (21) agencée sur un côté peau de l'utilisateur ;
une deuxième feuille (22) agencée sur un côté non-peau de l'utilisateur ; et
une feuille séparée (23) qui est différente de la première feuille (21) et de la deuxième feuille (22),
la découpe (20) est formée par pénétration de la première feuille (21) et de la deuxième feuille (22), et
la feuille séparée (23) comprend une région qui chevauche la région de bord (20a), et dans laquelle
la feuille séparée (23) est une feuille à dessin qui est agencée entre la première feuille (21) et la deuxième feuille (22), et
une région (23a) dans la feuille séparée (23) qui chevauche la région de bord (20a) est colorée d'une couleur différente de la couleur extérieure de la région de bord (20a).

2. Couche jetable de type culotte (11) selon la revendication 1, dans laquelle
la découpe (20) est formée par pénétration de la première feuille (21), de la deuxième feuille (22) et de la feuille séparée (23), et
une distance (d) depuis une extrémité supérieure de la partie au niveau de la taille côté abdomen (2) jusqu'à une extrémité supérieure de la feuille séparée (23) est inférieure ou égale à une longueur maximum (D) de la découpe (20) dans la direction allant de haut en bas.

3. Couche jetable de type culotte (11) selon la revendication 2, dans laquelle
une extrémité supérieure de la feuille séparée (23) est agencée de manière à chevaucher une extrémité supérieure de la première feuille (21) et une extrémité supérieure de la deuxième feuille (22), et une longueur (H1) de la feuille séparée (23) dans la direction allant de haut en bas est supérieure à une longueur maximum (D) de la découpe (20) dans la direction allant de haut en bas.

4. Couche jetable de type culotte (11) selon l'une quelconque des revendications 1 à 3, dans laquelle
ladite une partie d'extrémité (4a) du corps principal absorbant (4) comprend une région (42c, 43c) qui chevauche la région de bord (20a).

5. Couche jetable de type culotte (11) selon la revendication 4, dans laquelle
le corps principal absorbant (4) comprend :
un corps absorbant (41) qui absorbe et retient du liquide ;
une feuille de dessus (42) agencée sur un côté peau par rapport au corps absorbant (41) ; et
une feuille de support (43) agencée sur un côté non-peau par rapport au corps absorbant (41), et
une partie d'extrémité de la feuille de support (43) comprend une région (43c) qui chevauche la région de bord (20a).

6. Couche jetable de type culotte (11) selon la revendication 4 ou la revendication 5, dans laquelle
le corps principal absorbant (4) comprend :
un corps absorbant (41) qui absorbe et retient du liquide ;
une feuille de dessus (42) agencée sur un côté peau par rapport au corps absorbant (41) ; et
une feuille de support (43) agencée sur un côté non-peau par rapport au corps absorbant (41), et
une partie d'extrémité de la feuille de dessus (42) comprend une région (42c) qui chevauche la région de bord (20a).

7. Couche jetable de type culotte (11) selon l'une quelconque des revendications 1 à 6, dans laquelle,
des parties formant revers latéraux (430) sont mises en œuvre des deux côtés du corps principal absorbant (4) dans la direction allant dans le sens de la largeur le long du corps principal absorbant (4), et
une partie d'extrémité supérieure de chacune des parties formant revers latéraux (430) comprend une région (430a) qui chevauche la région de bord (20a).

8. Couche jetable de type culotte (11) selon la revendication 7, dans laquelle
le corps principal absorbant (4) comprend :
un corps absorbant (41) qui absorbe et retient du liquide ;
une feuille de dessus (42) agencée sur un côté peau par rapport au corps absorbant (41) ; et
une feuille de support (43) agencée sur un côté non-peau par rapport au corps absorbant (41), et
les parties formant revers latéraux (430) sont formées en permettant aux deux parties d'extrémité de la feuille de support (43) dans la direction allant dans le sens de la largeur d'être repliées vers l'arrière dans la direction allant dans le sens de la largeur et d'être assemblées avec la feuille de dessus (42).

9. Couche jetable de type culotte (11) selon l'une quelconque des revendications 1 à 8, dans laquelle
la partie au niveau de la taille côté abdomen (2) comprend une partie repliée vers l'arrière (210) dans laquelle une partie d'extrémité supérieure de la partie au niveau de la taille côté abdomen (2) est repliée vers l'arrière vers le bas, et
la partie repliée vers l'arrière (210) comprend une région qui chevauche une région de bord (20a).

10. Couche jetable de type culotte (11) selon la revendication 9, dans laquelle
une longueur de la partie repliée vers l'arrière (210) qui est repliée vers l'arrière vers le bas est plus longue qu'une longueur maximum (D) de la découpe (20) dans la direction allant de haut en bas.
